# EUROPEAN PATENT APPLICATION

(11) **EP 2 000 531 A1**
(43) Date of publication of application: **10.12.2008**
(21) Application number: 07450104.0
(22) Date of filing: 06.06.2007
(51) Int. Cl.: C12N 5/06, C12N 15/86

(54) **Antigen presenting cells**

(71) Applicant: BIOMAY AG, 1090 Wien (AT)
(72) Inventor: Baranyi, Ulrike, 1090 Vienna (AT); Linhart, Birgit, 1090 Vienna (AT); Pilat, Nina, 1090 Vienna (AT); Bagely, Jessamyn, Boston, MA 02115 (US); Iacomini, John, Boston, MA 02115 (US); Valenta, Rudolf, 1090 Vienna (AT); Wekerle, Thomas, 1090 Vienna (AT)
(74) Representative: Sonn & Partner Patentanwälte

(57) **Abstract**

The present invention relates to a method for inducing specific long-lasting robust immunological tolerance towards at least one polypeptide derived from at least one allergen by transplanting a hematopoietic (stem) cell which is produced to display the said at least one polypeptide derived from at least one allergen.

SEQUENCE LISTING

<110>  BIOMAY AG

<120>  Antigen presenting cells

<130>  R 48663

<160>  12

<170>  PatentIn version 3.4

<210>  1
<211>  27
<212>  DNA
<213>  Artificial

<220>
<223>  Primer

<400>  1
ggcgccatgg agacagacac actcctg                                          27

<210>  2
<211>  27
<212>  DNA
<213>  Artificial

<220>
<223>  Primer

<400>  2
gtaaccgaga tcggcgtcac cagtgga                                          27

<210>  3
<211>  24
<212>  DNA
<213>  Artificial

<220>
<223>  Primer

<400>  3
actggtgacg ccgatctcgg ttac                                             24

<210>  4
<211>  27
<212>  DNA
<213>  Artificial

<220>
<223>  Primer

<400>  4
gccggatcct cagactttgt agccacc                                          27

<210>  5
<211>  24
<212>  DNA
<213>  Artificial

<220>
<223>  Primer

<400>  5
gcccacagcg actttgtagc cacc                                             24

<210>  6
<211>  18
<212>  DNA
<213>  Artificial

<220>
<223>  Primer

<400>  6
tacaaagtcg ctgtgggc                                                    18


<210>  7
<211>  26
<212>  DNA
<213>  Artificial

<220>
<223>  Primer

<400>  7
ggcggatcct aacgtggctt cttctg                                           26


<210>  8
<211>  18
<212>  DNA
<213>  Artificial

<220>
<223>  Primer

<400>  8
cttgctcacg tcaccagt                                                    18


<210>  9
<211>  24
<212>  DNA
<213>  Artificial

<220>
<223>  Primer

<400>  9
actggtgacg tgagcaaggg cgag                                             24


<210>  10
<211>  24
<212>  DNA
<213>  Artificial

<220>
<223>  Primer

<400>  10
ggcagatctc taacgtggct tctt                                             24


<210>  11
<211>  24
<212>  DNA
<213>  Artificial

<220>
<223>  Primer

<400>  11
gcccacagct ctagatccgg tgaa                                              24


<210>  12
<211>  18
<212>  DNA
<213>  Artificial

<220>
<223>  Primer

<400>  12
ggatctagag ctgtgggc                                                     18

## Description

The present invention relates to a method for introducing allergens and derivatives thereof and means for preventing and treating an allergy or hypersensitivity (Coombs RRA, Gell PGH, Clinical Aspects of Immunology, chapter 13. Oxford: Blackwell Science, 1997; 23-25).

Allergy is the inherited or acquired specific alternation of the reaction capability against foreign (i.e. non-self) substances which are normally harmless ("allergens"). An allergy is connected with inflammatory reactions in the affected organ systems (skin, conjunctiva, nose, pharynx, bronchial mucosa, gastrointestinal tract), immediate disease symptoms, such as allergic rhinitis, conjunctivitis, dermatitis, anaphylactic shock and asthma, and chronic disease manifestations, such as late stage reactions in asthma and atopic dermatitis.

Type I allergy represents a genetically determined hypersensitivity disease which affects about 20% of the industrialised world population. The pathophysiological hallmark of Type I allergy is the production of immunoglobulin E (IgE) antibodies against otherwise harmless antigens (allergens).

Currently, the only causative form of allergy treatment is an allergen-specific immunotherapy, wherein increasing allergen doses are administered to the patient in order to induce allergen-specific unresponsiveness. This strategy has, however, limitations, as evidenced, for instance, by only partial effectiveness and by the risk of substantial side effects. Therefore, improved strategies for the treatment and prevention are an unmet goal.

The major disadvantage of allergen-specific immunotherapy is the dependency on the use of natural allergen extracts which are difficult, if not impossible to standardise, at least to an industrial production level. Such natural allergen extracts consist of different allergenic and non-allergenic compounds and due to this fact, it is possible that certain allergens are not present in the administered extract or - even worse - that patients can develop new IgE-specificities to components in the course of the treatment. Another disadvantage of extract-based therapy results from the fact that the administration of biologically active allergen preparations can induce anaphylactic side effects.

The application of molecular biology techniques in the field of allergen characterisation has allowed to isolate the cDNAs which code for all relevant environmental allergens and allowed the production of recombinant allergens. Using such recombinant allergens has made it possible to determine the individual patient's reactivity profile either by in vitro diagnostic methods (i.e. detection of allergen-specific IgE antibodies in serum) or by in vivo testing. Based on this technology, the possibility to develop novel component-based vaccination strategies against allergy, especially against Type I allergy, which are tailored to the patient's sensitisation profile, appeared to be possible. However, due to the similarity of the recombinant allergens to their natural counterparts, also recombinant allergens exhibit significant allergenic activity. Since the recombinant allergens closely mimick the allergenic activity of the wild type allergens, all the drawbacks connected with this allergenic activity in immunotherapy applying natural allergens are also present in recombinant allergens. In order to improve immunotherapy, the allergenic activity of recombinant allergens has to be reduced so that the dose of the administered allergens can be increased with only a low risk of anaphylactic side effects.

Another suggestion was to produce hypoallergenic allergen versions by genetic engineering based on the observation that allergens can naturally occur as isoforms which differ in only a few amino acid residues and/or in conformations with low IgE binding capacity. For example, oligomerisation of the major birch pollen allergen, Bet v 1, by genetic engineering yielded a recombinant trimer with greatly reduced allergenic activity. Alternatively, introduction of point mutations has been suggested to either lead to conformational changes in the allergen structure and thus disrupt discontinuous IgE epitopes or directly affect the IgE binding capacity (Valenta et al., Biol.Chem.380 (1999), 815-824).

It has also been shown that fragmentation of the allergen into few parts (e.g. into two parts) leads to an almost complete loss of IgE binding capacity and allergenic activity of the allergen due to a loss of their native-like folds (Vrtala et al. (J.Clin.Invest.99 (1997), 1673-1681) for Bet v 1, Twardosz et al. (BBRC 239 (1997), 197-204) for Bet v 4, Hayek et al. (J. Immunol.161 (1998), 7031-7039) for Aln g 4, Zeiler et al. (J.Allergy Clin. Immunol.100 (1997), 721-727) for bovine dander allergen, Elfman (Int.Arch.Allergy Immunol.117 (1998), 167-173) for Lep d2), Westritschnig (J.Immunol.172 (2004), 5684-5692) for Phlp 7),...). Fragmentation of proteins containing primarily discontinuous/conformational IgE epitopes leads to a substantial reduction of the allergen's IgE binding capacity. Based on this knowledge, it has been investigated in the prior art whether such hypoallergenic allergen fragments can induce protective immune responses in vivo (Westritschnig et al. (Curr. Opinion in Allergy and Clin. Immunol. 3 (2003), 495-500)).

Currently, immunotherapy is basically performed by administering allergens and derivatives thereof having a reduced IgE binding capacity orally, mucosally, subcutaneously or intravenously. Immunotherapy is an active vaccination treatment inducing an IgG response. This approach is limited by several shortcomings, as for instance its ineffectiveness in completely abolishing an allergic response and the restricted duration of its effect.

It is an object of the present invention to provide means and methods for an improved strategy for the prevention and treatment of an allergy based on a completely different strategy than conventional immunotherapy. Another object of the present invention is to provide means which allow a nearly life-long protection against development of allergic reactions.

Therefore, the present invention relates to a method for producing a hematopoietic cell expressing at least one polypeptide derived from at least one allergen by introducing nucleic acids or a DNA molecule which encodes said at least one polypeptide. Said nucleic acids or DNA molecule are preferably a DNA vector or another nucleic acid transferring vehicle harbouring a nucleic acid or DNA stretch encoding said at least one polypeptide. The at least one polypeptide may not only be introduced into the cell by transferring a cell therein but also be anchored on a hematopoietic cell. The method of the present invention is preferably performed in vitro.

Hematopoietic cells can advantageously be used in preventing and treating allergies when they are modified in a way to allow the production of polypeptides derived from allergens. Hematopoietic cells according to the present invention are capable of producing and secreting said polypeptides which, exposed to the immune system, induce specific immunological tolerance towards the said polypeptide at the T cell, B cell and effector cell levels. A further advantage is that hematopoietic cells can be transplanted or re-transplanted after modification (autologous hematopoietic cells) to an individual.

The mature or immature hematopoietic cells can be isolated from an individual with methods known in the art. It is especially preferred to obtain hematopoietic stem cells or hematopoietic progenitor cells from bone marrow, umbilical cord blood or from G-CSF (granulocyte-stimulating factor)-mobilized pe-riphal blood (Shizuru, Negrin and Weissman, Annu Rev Med 56 (2005):509-38; Copelan E.A., N Engl J Med 354 (17):1813-1826).

In the field of organ transplantation, induction of hematopoietic chimerism has been investigated as an approach for inducing tolerance to allo-antigens (Wekerle and Sykes, Annu Rev Med 2001; 52:353-70). In this scenario hematopoietic cells of the donor (e.g. in the form of bone marrow) are transplanted into an appropriately prepared recipient who also receives an organ graft from the same donor. Hematopoietic donor cells are long-term persistent, rendering the recipient chimeric and specifically tolerant towards the allogeneic donor. Alternatively, the recipient's own hematopoietic cells are modified in vitro to express a donor MHC allo-antigen (Madsen et al. Nature 1988, 332:161-164; Iacomini et al. Blood 99: 4394-4399, 2002; Sonntag KC et al. J Clin Invest 107: 65-71, 2001). Subsequently, the modified cells are transplanted back into the same individual, inducing so-called molecular chimerism and tolerance towards the single donor allo-antigen. This approach has also been applied to treat autoimmune disease in a mouse model for type I diabetes or multiple sclerosis (Steptoe et al. J Clin Invest 111:1357-1363, 2003; Xu et al. Mol Ther 13:42-48, 2006).

A type I allergic reaction is distinct from allo-immunity and autoimmune diseases. These separate entities differ in numerous immunological characteristics, including separate causes and triggers and separate types of effector cells and effector mechanisms. Besides, allergy is a completely separate and discrete clinical disorder, which is consequently treated with different therapies. Before the present invention, transplantation of autologous hematopoietic stem cells modified to present allergen has not been investigated or used in relation to allergy.

According to the present invention "polypeptide derived from at least one allergen" ("allergen derivative") means that a DNA region of the DNA vector encodes for at least one polypeptide which is an allergen or a fragment or derivative thereof. Allergen derivatives include in particular derivatives of a wild-type allergen showing a significantly reduced allergenic activity compared to that of the wild-type allergen. The allergenic activity can be impaired by several means which all aim at destabilising the protein forms by disrupting its intramolecular disulphide bridges, thereby destabilising its three-dimensional structure. These derivatives have a reduced allergenicity while still retaining T cell reactivity, thus being more suitable for systemic administration of high doses of the immunogen which induces in particular allergen specific IgG which compete with IgE for the binding to the wild-type allergen. Hence, hypoallergenic derivatives are suitable for therapeutic and prophylactic purposes.

The production of allergen derivatives, in particular of hypoallergen polypeptides, may be performed by introducing mutations into the native sequence. This may be achieved by: introducing substitutions, deletions or additions in or by altering the three dimensional structure of the protein such that the three-dimensional conformation of the protein is lost. This may be achieved, amongst others, by expressing the protein in fragments or by deleting cysteine residues involved in disulphide bridge formation or by deleting or adding residues such that the tertiary structure of the protein is substantially altered.

Hypoallergenic allergens are preferably mutated to decrease or abolish its IgE-binding reactivity and/or histamine release activity, whilst retaining its T cell reactivity and/or the ability to stimulate an immune response against the wild-type allergen. The allergenic activity, and consequently the reduction in allergenic activity, of the mutant allergens may be compared to the wild type by any of the following methods: histamine release activity or by IgE-binding reactivity. "Substantially reduced allergenic activity" means that the allergenic activity as measured by residual IgE-binding activity is reduced to a maximum of 50% of the activity of the native-unmodified or non-mutated protein, preferably to a maximum of 20%, more preferably to a maximum of 10%, still more preferably to a maximum of 5%, still more preferably to less than 5%. Alternatively, "substantially" also means that the histamine release activity of the mutant is reduced by at least a 100-fold factor as compared to the native protein, preferably by a factor of 1000-fold, still more preferably by a factor of 10000-fold.

An allergen derivative according to the present invention may also be a reshuffled or mosaic polypeptide obtainable by fusing together fragments of at least one allergen, preferably wild-type allergen, in a non-natural order (see e.g. WO 2004/065414).

The term "polypeptide" as used herein refers to molecules comprising at least 5 amino acid residues (preferably at least 7, 8 or 10) covalently bound to each other via peptide bonds.

As used herein, "at least one polypeptide derived from at least one allergen" means that said polypeptide comprises the entire amino acid sequence of an allergen or fragments thereof.

The fragments of the allergen comprise at least 10, preferably at least 15 successive amino acids of the at least one allergen. Furthermore, the at least one polypeptide derived from at least one allergen shows at least 80% (preferably at least 90%, more preferably at least 95%, in particular 100%) identity with the relevant sequence or sequence fragment of the allergen.

Of course, the polypeptide may comprise more than one allergen or fragments thereof fused together, whereby it is preferred that the polypeptide does not bind to IgE.

According to the present invention "identity" ("identical") is determined by comparing two optimally aligned sequences over a comparison window, where the fragment of the amino acid sequence in the comparison window may comprise additions or deletions (e.g., gaps or overhangs) as compared to the reference sequence (which does not comprise additions or deletions) for optimal alignment of the two sequences. In general, sequences are aligned so that the highest order match is obtained (see, e.g.: Computational Molecular Biology, Lesk, A.M. , ed., Oxford University Press, New York,1988 ; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G. , Academic Press, 1987; and Sequence Analysis Primer, Gribskov, M. and Devereux, J., eds., M Stockton Press, New York,1991; Carillo et al. (1988) SIAM J Applied Math 48:1073).

Whether any two amino acid molecules have amino sequences that are at least, for example, 80%, 85%, 90%, 95%, 96%, 97%, 98% or 99% "identical", can be determined using known computer algorithms such as the "FAST A" program, using for example, the default parameters as in Pearson et al. (1988) PNAS USA 85: 2444 (other programs include the GCG program package (Devereux, J., et al., Nucleic Acids Research (1984) Nucleic Acids Res., 12, 387-395), BLASTP, BLASTN, FASTA (Altschul, S.F., et al., J Molec Biol 215: 403 (1990); Guide to Huge Computers, Martin J. Bishop, ed., Academic Press, San Diego, 1994, and Carilloet al, (1988)SIAM J Applied Math 48 : 1073). For instance, the BLAST tool of the NCBI database can be used to determine identity. Other commercially or publicly available programs include, DNAS-tar "MegAlign" program (Madison,WI) and the University of Wisconsin Genetics Computer Group(UWG) "Gap" program (Madison Wu)). Percent identity of proteins and/or peptides can be determined, for example, by comparing sequence information using a GAP computer program (e.g. Needleman et al., (1970) J. Mol. Biol. 48:443, as revised by Smith and Waterman (1981) Adv. Appl. Math. 2:482). Briefly, the GAP program defines similarity as the number of aligned symbols (i.e., nucleotides or amino acids) which are similar, divided by the total number of symbols in the shorter of the two sequences. Default parameters for the GAP program can include : (1) a unary comparison matrix (containing a value of 1 for identities and for non-identities) and the weighted comparison matrix of Gribskov et al. 14:6745, as described by Schwartz and Dayhoff, eds., ATLAS OF PROTEIN SEQUENCE AND STRUCTURE, National Biomedical Research Foundation, pp. 353-358 (1979); (2) a penalty of 3.0 for each gap and an additional 0.10 penalty for each symbol in each gap; and (3) no penalty for end gaps.

As used herein, the term "at least 80% identical to" refers to percent identities from 80 to 99.99 relative to the reference allergens. Consequently, the polypeptide of the present invention may also comprise one or more amino acid modifications (i.e. substitutions, deletions, insertions).

Identity at a level of 80% or more is indicative of the fact that, assuming for exemplification purposes a test and reference polypeptide length of 100 amino acids is compared, no more than 20% (i.e. 20 out of 100) of amino acid residues in the test polypeptide differ from that of the reference polypeptide. Such differences can be represented as point mutations randomly distributed over the entire length of an amino acid sequence or they can be clustered in one or more locations of varying length up to the maximum allowable, e.g. 20/100 amino acid difference (approx. 80% identity). Differences are defined as amino acid substitutions, insertions or deletions. At the level of homologies or identities above about 85-90%, the result should be independent of the program and gap parameters set; such high levels of identity can be assessed readily, often without relying on software.

By the term "DNA vector" as used herein, a DNA vehicle is intended which is derived from viral or non-viral, e.g., bacterial, species that has been designed to encode an exogenous or heterologous nucleic acid sequence. Thus, the term includes conventional bacterial plasmids. Such plasmids or vectors can include plasmid sequences from viruses or phages. Such vectors include chromosomal, episomal and virus-derived vectors, e.g., vectors derived from bacterial plasmids, bacteriophages, yeast episomes, yeast chromosomal elements, and viruses (e.g. retroviruses). Vectors may also be derived from combinations thereof, such as those derived from plasmid, viral and bacteriophage eukaryotic or prokaryotic genetic elements, cosmids, and phagemids. The term also includes non-replicating viruses that transfer a gene from one cell to another. The term should also be construed to include non-plasmid and non-viral compounds which facilitate transfer of nucleic acid into cells, such as, for example, polylysine compounds and the like. Therefore, the term "DNA vector" as used herein, can be used interchangeable with the terms "DNA vehicle", "virus", "retrovirus" etc..

The DNA vector may comprise transcriptional regulatory elements (or DNA regulatory elements) which, when operably linked to a gene of interest, are capable of altering the transcription of such gene of interest in a specific way which is characteristic of such element. Transcriptional regulatory elements include promoters, enhancers, suppressors, transcriptional start sites, transcriptional stop sites, polyadenylation sites, and the like.

To transform a hematopoietic cell with a DNA vector or DNA molecules according to the present invention, several systems are available, depending upon whether it is desired to insert the heterologous protein DNA construct into the host cell chromosomal DNA, or to allow it to exist in an extrachromosomal form.

If the heterologous protein's DNA sequence and an operably linked promoter is introduced into the hematopoietic cell as non-replicating DNA (or RNA) molecule which may either be a linear molecule or, more preferably, a closed covalent circular molecule that is incapable of autonomous replication, the expression of the heterologous protein may occur through the transient expression of the introduced sequence.

Genetically stable transformants may be constructed with vector systems or transformation systems, whereby the heterologous protein's DNA is integrated into the host chromosome. Such integration may occur de novo within the cell or be assisted by transformation with a vector that functionally inserts itself into the host chromosome. Vectors capable of chromosomal insertion include, for example, retroviral vectors, transposons or other DNA elements which promote integration of DNA sequences in chromosomes, especially DNA sequence homologous to a desired chromosomal insertion site.

Cells that have stably integrated the introduced DNA into their chromosomes are selected by also introducing one or more markers that allow for selection of host cells with the desired sequence. For example, the marker may provide biocide resistance, e.g., resistance to antibiotics or heavy metals, such as copper, or the like. The selectable marker gene can either be directly linked to the DNA gene sequences to be expressed or introduced into the same cell by co-transfection.

In another preferred embodiment, the introduced sequence is incorporated into a plasmid or viral vector capable of autonomous replication in the recipient host. Any of a wide variety of vectors may be employed for this purpose, as outlined below.

Important factors in selecting a particular plasmid or viral vector include the ease with which recipient cells that contain the vector may be recognised and selected from those recipient cells which do not contain the vector; the number of copies of the vector which are desired in a particular host; and whether it is desirable to be able to "shuttle" the vector between host cells of different species.

Preferred eukaryotic plasmids include those derived from the bovine papilloma virus, vaccinia virus, and SV40. Such plasmids are well known in the art and are commonly or commercially available. For example, mammalian expression vector systems in which it is possible to cotransfect with a helper virus to amplify plasmid copy number, and integrate the plasmid into the chromosomes of host cells have been described (Perkins, A. S. et al., Mol. Cell Biol. 3:1123 (1983); Clontech, USA).

Once the vector or DNA sequence containing the construct(s) has been prepared for expression, the DNA construct(s) is introduced into the hematopoietic cell by any of a variety of suitable means, including transfection, electroporation or delivery by liposomes. DEAE-dextran or calcium phosphate, may be useful in the transfection protocol.

After the introduction of the vector in vitro, recipient cells are grown in a selective medium, that is, medium that selects for the growth of vector-containing cells. Expression of the cloned gene sequence(s) result(s) in the production of the heterologous protein.

According to a preferred embodiment of the present invention the hematopoietic cell is selected from the group consisting of monocyte, macrophage, neutrophil, basophil, hemopoietic stem cell, eosinophil, T-cell, B-cell, NK-cell and dendritic cell.

The DNA vector comprising a nucleic acid molecule which encodes for an allergen or a fragment or derivative thereof may be introduced into all kinds of hematopoietic cells listed above. All these cells are capable of expressing and secreting a polypeptide derived from an allergen.

The DNA vector is preferably a viral, preferably retroviral, or a plasmid vector (Papapetrou EP et al., Gene Therapy 12 (2005):118-130).

According to a preferred embodiment of the present invention the vector is transiently introduced in the hematopoietic cell.

The transient introduction of the vector into the cells allows that over time the hematopoietic cells will lose the DNA encoding for the allergen derivative. This is particularly advantageous when the expression of the allergen derivative is only required for a limited period of time. For instance, transfected hematopoietic cells can be administered or transplanted to an individual without the risk that said individual will become chimeric and produces allergen derivatives throughout his/her life.

The method according to the present invention may be performed with polypeptides derived from any kind of allergen, wherein the allergen is preferably selected from the group consisting of Amb a 1, Amb a 2, Amb a 3, Amb a 5, Amb a 6, Amb a 7, Amb a 8, Amb a 9, Amb a 10, Amb t 5, Art v 1, Art v 2, Art v 3, Art v 4, Art v 5, Art v 6, Hel a 1, Hel a 2, Hel a 3, Mer a 1, Che a 1, Che a 2, Che a 3, Sal k 1, Cat r 1, Pla 1 1, Hum j 1, Par j 1, Par j 2, Par j 3, Par o 1, Cyn d 1, Cyn d 7, Cyn d 12, Cyn d 15, Cyn d 22w, Cyn d 23, Cyn d 24, Dac g 1, Dac g 2, Dac g 3, Dac g 5, Fes p 4w, Hol 1 1, Lol p 1, Lol p 2, Lol p 3, Lol p 5, Lol p 11, Pha a 1, Phl p 1, Phl p 2, Phl p 4, Phl p 5, Phl p 6, Phl p 11, Phl p 12, Phl p 13, Poa p 1, Poa p 5, Sor h 1, Pho d 2, Aln g 1, Bet v 1, Bet v 2, Bet v 3, Bet v 4, Bet v 6, Bet v 7, Car b 1, Cas s 1, Cas s 5, Cas s 8, Cor a 1, Cor a 2, Cor a 8, Cor a 9, Cor a 10, Cor a 11, Que a 1, Fra e 1, Lig v 1, Ole e 1, Ole e 2, Ole e 3, Ole e 4, Ole e 5, Ole e 6, Ole e 7, Ole e 8, Ole e 9, Ole e 10, Syr v 1, Cry j 1, Cry j 2, Cup a 1, Cup s 1, Cup s 3w, Jun a 1, Jun a 2, Jun a 3, Jun o 4, Jun s 1, Jun v 1, Pla a 1, Pla a 2, Pla a 3, Aca s 13, Blo t 1, Blo t 3, Blo t 4, Blo t 5, Blo t 6, Blo t 10, Blo t 11, Blo t 12, Blo t 13, Blo t 19, Der f 1, Der f 2, Der f 3, Der f 7, Der f 10, Der f 11, Der f 14, Der f 15, Der f 16, Der f 17, Der f 18w, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Eur m 2, Eur m 14, Gly d 2,Lep d 1, Lep d 2, Lep d 5, Lep d 7, Lep d 10, Lep d 13, Tyr p 2, Tyr p 13, Bos d 2, Bos d 3, Bos d 4, Bos d 5, Bos d 6, Bos d 7, Bos d 8, Can f 1, Can f 2, Can f 3, Can f 4, Equ c 1, Equ c 2, Equ c 3, Equ c 4, Equ c 5, Fel d 1, Fel d 2, Fel d 3, Fel d 4, Fel d 5w, Fel d 6w, Fel d 7w, Cav p 1, Cav p 2, Mus m 1, Rat n 1, Alt a 1, Alt a 3, Alt a 4, Alt a 5, Alt a 6, Alt a 7, Alt a 8, Alt a 10, Alt a 12, Alt a 13, Cla h 2, Cla h 5, Cla h 6, Cla h 7, Cla h 8, Cla h 9, Cla h 10, Cla h 12, Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22w, Asp f 23, Asp f 27, Asp f 28, Asp f 29, Asp n 14, Asp n 18, Asp n 25, Asp o 13, Asp o 21, Pen b 13, Pen b 26, Pen ch 13, Pen ch 18, Pen ch 20, Pen c 3, Pen c 13, Pen c 19, Pen c 22w, Pen c 24, Pen o 18, Fus c 1, Fus c 2, Tri r 2, Tri r 4, Tri t 1, Tri t 4, Cand a 1, Cand a 3, Cand b 2, Psi c 1, Psi c 2, Cop c 1, Cop c 2, Cop c 3, Cop c 5, Cop c 7, Rho m 1, Rho m 2, Mala f 2, Mala f 3, Mala f 4, Mala s 1, Mala s 5, Mala s 6, Mala s 7, Mala s 8, Mala s 9, Mala s 10, Mala s 11, Mala s 12, Mala s 13, Epi p 1, Aed a 1, Aed a 2, Api m 1, Api m 2, Api m 4, Api m 6, Api m 7, Bom p 1,Bom p 4, Bla g 1, Bla g 2, Bla g 4, Bla g 5, Bla g 6, Bla g 7, Bla g 8, Per a 1, Per a 3, Per a 6, Per a 7, Chi k 10, Chi t 1-9, Chi t 1.01, Chi t 1.02, Chi t 2.0101, Chi t 2.0102, Chi t 3, Chi t 4, Chi t 5, Chi t 6.01, Chi t 6.02, Chi t 7, Chi t 8, Chi t 9, Cte f 1, Cte f 2, Cte f 3, Tha p 1, Lep s 1, Dol m 1, Dol m 2, Dol m 5, Dol a 5, Pol a 1, Pol a 2, Pol a 5, Pol d 1, Pol d 4, Pol d 5, Pol e 1, Pol e 5, Pol f 5, Pol g 5, Pol m 5, Vesp c 1, Vesp c 5, Vesp m 1, Vesp m 5, Ves f 5, Ves g 5, Ves m 1, Ves m 2, Ves m 5, Ves p 5, Ves s 5, Ves vi 5, Ves v 1, Ves v 2, Ves v 5, Myr p 1, Myr p 2, Sol g 2, Sol g 4, Sol i 2, Sol i 3, Sol i 4, Sol s 2, Tria p 1, Gad c 1, Sal s 1, Bos d 4, Bos d 5, Bos d 6, Bos d 7, Bos d 8, Gal d 1, Gal d 2, Gal d 3, Gal d 4, Gal d 5, Met e 1, Pen a 1, Pen i 1, Pen m 1, Pen m 2, Tod p 1, Hel as 1, Hal m 1, Ran e 1, Ran e 2, Bra j 1, Bra n 1, Bra o 3, Bra r 1, Bra r 2, Hor v 15, Hor v 16, Hor v 17, Hor v 21, Sec c 20, Tri a 18, Tri a 19, Tri a 25, Tri a 26, Zea m 14, Zea m 25, Ory s 1, Api g 1, Api g 4, Api g 5, Dau c 1, Dau c 4, Cor a 1.04, Cor a 2, Cor a 8, Fra a 3, Fra a 4, Mal d 1, Mal d 2, Mal d 3, Mal d 4, Pyr c 1, Pyr c 4, Pyr c 5, Pers a 1, Pru ar 1, Pru ar 3, Pru av 1, Pru av 2, Pru av 3, Pru av 4, Pru d 3, Pru du 4, Pru p 3, Pru p 4, Aspa o 1, Cro s 1, Cro s 2, Lac s 1, Vit v 1, Mus xp 1, Ana c 1, Ana c 2, Cit 1 3, Cit s 1, Cit s 2, Cit s 3, Lit c 1, Sin a 1, Gly m 1, Gly m 2, Gly m 3, Gly m 4, Vig r 1, Ara h 1, Ara h 2, Ara h 3, Ara h 4, Ara h 5, Ara h 6, Ara h 7, Ara h 8, Len c 1, Len c 2, Pis s 1, Pis s 2, Act c 1, Act c 2, Cap a 1w, Cap a 2, Lyc e 1, Lyc e 2, Lyc e 3, Sola t 1, Sola t 2, Sola t 3, Sola t 4, Ber e 1, Ber e 2, Jug n 1, Jug n 2, Jug r 1, Jug r 2, Jug r 3, Ana o 1, Ana o 2, Ana o 3, Ric c 1, Ses i 1, Ses i 2, Ses i 3, Ses i 4, Ses i 5, Ses i 6, Cuc m 1, Cuc m 2, Cuc m 3, Ziz m 1, Ani s 1, Ani s 2, Ani s 3, Ani s 4, Arg r, Asc s 1, Car p 1, Den n 1, Hev b 1, Hev b 2, Hev b 3, Hev b 4, Hev b 5, Hev b 6.01, Hev b 6.02, Hev b 6.03, Hev b 7.01, Hev b 7.02, Hev b 8, Hev b 9, Hev b 10, Hev b 11, Hev b 12, Hev b 13, Hom s 1, Hom s 2, Hom s 3, Hom s 4, Hom s 5 and Trip s 1.

Particularly preferred allergens are Phl p 1, Phl p 2, Phl p 5, Phl p 6, Der p 1, Der p 2, Der p 5, Der p 7, Der p 21, Fel d 1, Bet v 1, Ole e 1, Par j 2, Can f 1 and Can f 2.

The allergen derivative used in the method according to the present invention is preferably hypoallergenic. Only hypoallergenic molecules guarantee a safe vaccination with a reduced risk of allergic reactions or other side effects.

The hematopoietic cell may secret the allergen derivative to the outside of the cell, so that the polypeptide will be exposed to the immune system. However, it is also advantageous to provide the allergen derivative with at least one transmembrane domain so that the hematopoietic cell itself displays the allergen derivative on its surface to the immune system. Therefore, it is preferred that the at least one polypeptide is fused to a signal sequence and/or transmembrane domain.

However, it is, of course, also possible to fuse the allergen derivative to another peptide or polypeptide, which may also have immunostimulatory or therapeutic effects.

The DNA vector, DNA vehicle, polypeptide anchored vehicle or virus is preferably introduced into the hematopoietic cell by chemical methods, preferably by using cationic lipids and cationic polymers, physical methods, preferably by particle bombardment, micro-injection and electroporation, or viral methods by interaction of the viral envelope with cell surface receptors or abundant phospholipids.

Another aspect of the present invention relates to a mammalian viral vector DNA comprising at least one nucleic acid molecule which encodes for a polypeptide derived from an allergen.

Viral vectors and vehicles are regularly used for infecting mammalian cells and transducing DNA molecules encoding for allergen derivatives. These vectors may additionally harbour vector elements which are required for the successful transduction of coding DNA molecules into the cells. Moloney murine leukaemia based retroviral vectors or HIV-1 based lentiviral vectors are commonly used for mammalian cell infections (Sinn,PL et al., Gene Therapy 12 (2005):1089-1098).

The vector preferably comprises long terminal repeats (LTR's) of preferably moloney murine leukaemia retrovirus or long terminal repeat promoter-enhancer elements of myeloproliferative sarcoma virus (MPSV). Alternatively long-term tissue or cell specific expression can be facilitated by the choice of promoters like albumin promoters or cytomegalovirus (CMV) promoters. Alternatively episomal vectors comprise e.g. origin of replication of EBV or SV40 or human chromosomal S/MAR.

According to a preferred embodiment of the present invention the allergen is selected from the group consisting of Phl p 1, Phl p 2, Phl p 5, Phl p 6, Der p 1, Der p 2, Der p 5, Der p 7, Der p 21, Fel d 1, Bet v 1, Ole e 1, Par j 2, Can f 1 and Can f 2.

The vector may comprise derivatives of any known allergen.

The allergen derivative is preferably hypoallergenic.

According to a further preferred embodiment of the present invention the at least one polypeptide is fused to a secretion signal sequence, membrane anchoring domain and/or transmembrane domain.

The at least one polypeptide is preferably fused to a secretion signal sequence, a membrane anchoring domain and/or a transmembrane domain. The fusion of the polypeptide of the present invention either to a secretion signal sequence alone or to a secretion signal sequence in combination with a membrane anchoring domain or a transmembrane domain allows to create a hematopoietic cell secreting at least one polypeptide of the present invention in the extracellular matrix or comprising at least one polypeptide of the present invention bound to the extracellular side of the cell membrane. Such cells are suitable to present to the immune system of an individual the polypeptide of the invention derived from an allergen. It is particularly advantageous to fuse the polypeptide of the invention to secretion signal sequences and a membrane anchoring domain or a transmembrane domain so that the polypeptide will be immobilised on the cell surface. This allows to expose the polypeptide to the immune system without releasing the polypeptide from the cell. This results in a cell which is much more efficient in establishing immunity against an allergen than conventional methods.

Secretion signal sequences, membrane anchoring domains and transmembrane domains preferably used are well known to the person skilled in the art. (Coloma et al., J Imm Methods 152 (1992): 89-104; Gronwald et al., Proc Natl Acad Sci USA 85 (1988): 3435-3439)

Yet another aspect of the present invention relates to a hematopoietic cell having a cell membrane and expressing at least one polypeptide derived from at least one allergen.

A membrane anchoring domain or a transmembrane domain is preferably integrated into such a cell membrane.

According to a preferred embodiment of the present invention the at least one polypeptide is bound to the extracellular side of the cell membrane.

According to another preferred embodiment of the present invention the hematopoietic cell comprises a mammalian viral vector DNA according to the present invention.

The hematopoietic cell is preferably selected from the group consisting of monocyte, macrophage, neutrophil, basophil, hemopoietic stem cell, eosinophil, T-cell, B-cell, NK-cell and dendritic cell.

Another aspect of the present invention relates to the use of a vector DNA or a host cell according to the present invention for manufacturing a medicament for the treatment or prevention of an allergy.

The medicament of the present invention can be administered to an individual suffering or at risk for suffering an allergy. It is preferred that the medicament of the present invention comprises autologous hematopoietic cells. This means that at the beginning of the treatment hematopoietic cells are isolated from an individual, transfected with the DNA vector of the present invention and administered to the same individual from whom the cells were obtained.

Another aspect of the present invention relates to a pharmaceutical formulation comprising a vector DNA or a host cell according to the present invention.

The present invention provides a strategy for the prevention and treatment of an allergy, which is characterised by the following steps:
- providing one or more allergens/foreign antigens, allergen/foreign antigen- derivatives, allergen/foreign antigen-derived epitopes/fragments,
- engineering said allergens/foreign antigens, allergen/foreign antigen-derivatives, allergen/foreign antigen-derived epitopes/fragments so that they are displayed on a self structure (e.g., hematopoietic cells) either by directly attaching the allergens/foreign antigens, allergen/foreign antigen-derivatives, allergen/foreign antigen-derived epitopes/fragments on the structure or by expressing the allergens/foreign antigens, allergen/foreign antigen-derivatives, allergen/foreign antigen-derived epitopes/fragments on the self structure,
- transplanting the selfstructure containing allergens/foreign antigens, allergen/foreign antigen-derivatives, allergen/foreign antigen-derived epitopes/fragments into an individual in order to induce specific non-responsiveness to the allergens/foreign antigens, allergen/foreign antigen-derivatives, allergen/foreign antigen-derived epitopes/fragments.

The present strategy leads to the presence of high numbers of allergen-bearing self structures (e.g. hematopoietic cells) in the recipient. The long-term existence of the modified self structures in the recipient seems critical for the success of this strategy.

The present strategy provides a way to achieve specific immunological unresponsiveness to one or more allergens while leaving all other physiological immune functions of the individual unperturbed. This so-called "state of tolerance" is especially robust as evidenced by the results of numerous assays. This entails the advantage that the tolerance achieved by the present strategy cannot be easily broken by physiological factors possibly occurring in any individual (e.g. infections). Another major advantage is that tolerance is very long-lasting, probably life-long. This strategy can be used for achieving tolerance to a single wild-type allergen, hybrid molecules (two or more allergens or allergen derivatives), hypoallergenic derivatives with retained T cell epitopes, T cell epitope containing peptides, or B cell epitope-derived peptides (reviewed in Linhart and Valenta. Curr Opin Immunol 17: 646-655, 2005).

The present strategy can be used in two scenarios: as preventive approach and as therapeutic approach. In both scenarios the strategy of the present invention is used to induce life-long allergen-specific tolerance.
- Preventive approach: The incidence of allergies in industrialised countries has surpassed 25% and is still rising. Furthermore, it is becoming possible with increasing precision to identify newborns who face a particularly high risk to develop severe allergies. Thus, newborns at increased risk, or eventually the general population at moderate risk, are potential groups of individuals who will benefit from strategies that prophylactically preventing the development of allergies. Sensitisation to allergens usually occurs within the first years after birth. Therefore, the ideal time frame for prophylactic intervention would be shortly after birth. One possibility would be to obtain immature hematopoietic cells (e.g. hematopoietic stem cells) from an individual (for instance, by harvesting and storing cord blood at birth, as is currently frequently done for other purposes). These cells, or subsets thereof, would be manipulated in vitro (under GMP conditions) by inserting or coupling the desired allergens/foreign antigens, allergen/foreign antigen-derivatives, allergen/foreign antigen-derived epitopes/fragments. These modified cells would then be re-introduced after appropriate preparation of the individual into the same individual (e.g. via intravenous infusion). Alternatively, mature cells or self-structures could be modified or used as vehicles for allergens/foreign antigens, allergen/foreign antigen-derivatives, allergen/foreign antigen-derived epitopes/fragments and re-introduced in the same individual like described above.
- Therapeutic approach: Patients of any age with established type I allergy, especially with severe or life-threatening subtypes, are candidates for using the strategy of the present invention in a therapeutic fashion. Hematopoietic cells, or other cell types, would be obtained from said individuals (for instance, by harvesting bone marrow or mobilised peripheral blood leukocytes, techniques which are clinically used for other purposes as a matter of routine). These cells, or subsets thereof, would be manipulated in vitro by genetic modification, inserting the desired allergens/foreign antigens, allergen/foreign antigen-derivatives, allergen/foreign antigen-derived epitopes/fragments. Alternatively, mature cells or self-structures (e.g. antibodies) could be modified or coupled with allergens or peptides. These modified cells or self structures would then be re-introduced after appropriate preparation of the individual into the same individual (e.g. via intravenous infusion or subcutaneous or oral application).

Permanent, robust tolerisation towards the transduced allergens is achieved with the present invention by inducing molecular chimerism (i.e. the presence of modified self-structures) through transplantation of hematopoietic cells transduced with allergens/foreign antigens, allergen/foreign antigen-derivatives, allergen/foreign antigen-derived epitopes/fragments,

According to preferred embodiment of the present invention, prevention of the development of allergen-specific antibodies (including the IgE subtype), prevention of release of allergy mediators, prevention of allergen-specific T cell reactivity and prevention of allergic skin reaction could be established in in vivo experiments in an animal model.

Tolerance towards an allergen has been achieved by molecular chimerism through the transplantation of syngeneic (genetically identical, corresponding to the autologous situation in the clinical setting) hematopoietic stem cells which express a recombinant allergen after genetic modification. In this murine model, tolerance ensues and prevents the development of type I allergy.

The present technology can be used as preventive as well as therapeutic approach.

For preventing type I allergy, individuals who were identified to be at high risk for developing severe allergies could be treated prophylactically with the present technology. Blood cells, for example, from stored cord blood cells could be purified to obtain progenitor cells (e.g. by sorting CD34 positive cells). The isolation of bone marrow cells and purification of CD34 positive progenitor cells could be an alternative. Isolated autologous cells or cells from genetically identical individuals could be cultured in the presence of cytokines and treated several times with allergen-introducing vehicles. These vehicles could be retroviral transgene-integrating recombinant particles or other allergen-introducing vehicles. Genetically modified cultivated cells contain or express, at least transiently, allergens/foreign antigens, allergen/foreign antigen-derivatives, allergen/foreign antigen-derived epitopes/fragments on the surface, or secrete these allergens.

The genetically modified cells are injected intravenously in appropriately pre-treated individuals. By expression of the allergen tolerance would be induced by recognising the allergen like a self protein. The individual expresses the introduced allergen on the surface of hematopoietic cells or secretes the introduced allergen. The presence of cells expressing the introduced allergen is known as molecular chimerism. Molecular chimerism leads to long-lasting tolerisation towards the introduced allergen. Therefore, even if the tolerised individual gets repeatedly in contact with allergens, type I allergy does not develop. A preferred method is shown in the example section below.

For treatment of allergic patients, autologous mobilised peripheral blood leukocytes, for example, stimulated with G-CSF (granulocyte colony stimulating factor) are isolated and collected. The collection of autologous bone marrow cells could be an alternative. Cells could be purified, for example CD34 positive progenitor cells. These cells could be cultivated ex vivo in the presence of cytokines and treated with allergen-introducing vehicles several times. The allergen expression is determined for example, by flow cytometry, and allergen-bearing cells are introduced into pretreated patients by intravenous infusion. Molecular chimerism can be monitored by expression of allergens in white blood cells.

The present invention is further described by the following examples and the figures, yet without being restricted thereto.

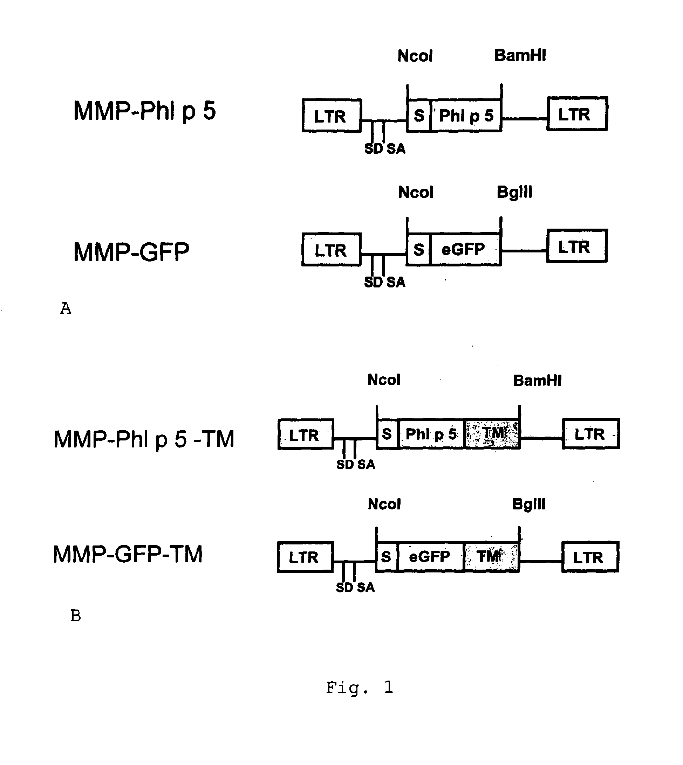

Fig. 1 shows a diagram of MMP-based retroviral vectors encoding Phl p 5 and eGFP fused to a signal sequence (S) for secreting proteins (A) and an additional transmembrane domain (TM) for membrane-anchored proteins (B). LTR, long-terminal repeat, SD, splicing donor, SA, splicing acceptor. Start codon was inserted with NcoI site. Stop codon was inserted directly before the BamHI or BglII site.

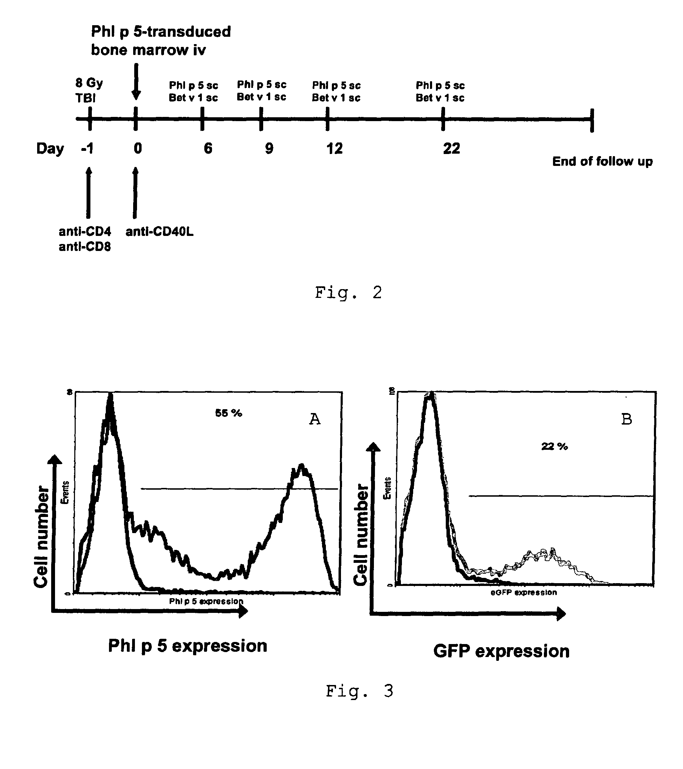

Fig. 2 shows an experimental protocol for the induction of allergen-specific tolerance in vivo. Balb/c recipients were pretreated with 8 Gy TBI (total body irridiation) and T cell-depleting antibodies (anti-CD4 and anti-CD8 monoclonal antibodies) and transplanted with allergen-transduced BM cells intravenously (iv). After transplantation of allergen-transduced BM, mice received an anti-CD40L mAb. Six, 9, 12 and 22 weeks after BMT, mice were injected subcutaneously (s.c.) 5 µg recombinant Phl p 5 and 5µg recombinant Bet v 1 (plus aluminumhydroxide). Before each immunisation, mice were bled for obtaining serum for further analyis. Thirty to 40 weeks after BMT, mice were sacrificed.

Fig. 3 shows the percentage of bone marrow cells expressing Phl p 5 (A) and eGFP (B) after retroviral transduction and cultivation. Bone marrow cells were tranduced with VSV-Phl p 5-TM, mock-transduced or VSV-eGFP-TM. Following transduction, mock-transduced bone marrow cells and Phl p 5-transduced cells were stained with Phl p 5-specific biotinylated antibody and phycoerythrin-labelled streptavidin (A), and were analysed by flow cytometry. Bone marrow cells transduced with VSV-eGFP-TM were analysed unstained and compared to untransduced bone marrow cells (B).

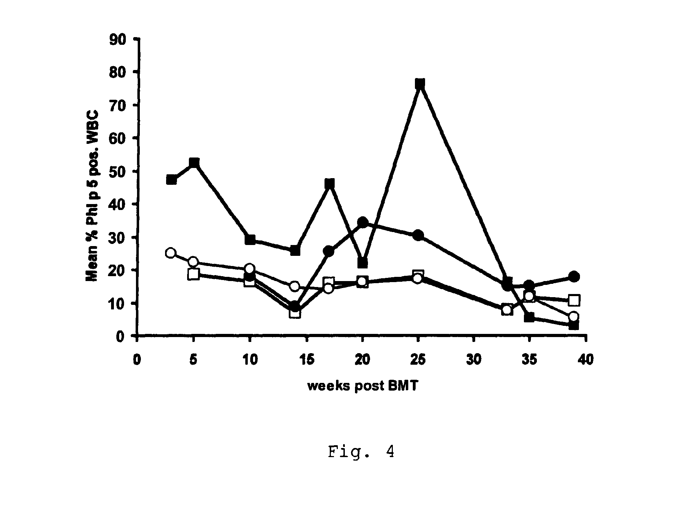

Fig. 4 shows the high levels of long-lasting stable Phl p 5 chimerism among various leukocyte lineages. The percentage of Phl p 5-positive cells among subsets of peripheral white blood cells (WBC) was determined by two-colour flow cytometry at multiple time points after BMT. Percentage chimerism is shown as mean (n=3). Peripheral blood leukocytes were stained with lineage-specific fluorescein isothiocyanate-conjugated antibodies and Phl p 5-specific antibody. Long-term persistence of substantial levels of multi-lineage molecular chimerism demonstrates that true hematopoietic stem cells have been successfully transduced with Phl p 5 in vitro. CD4 (□), CD8 (•), B (o) and monocytes, granulocytes (■) The percentage of Phl p 5 positive cells determined by two-colour flow cytometry.

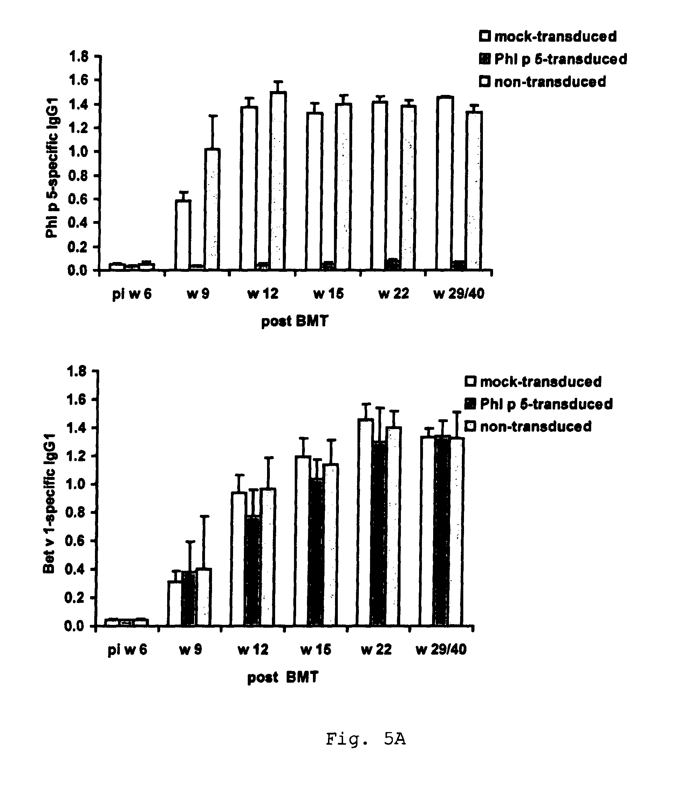

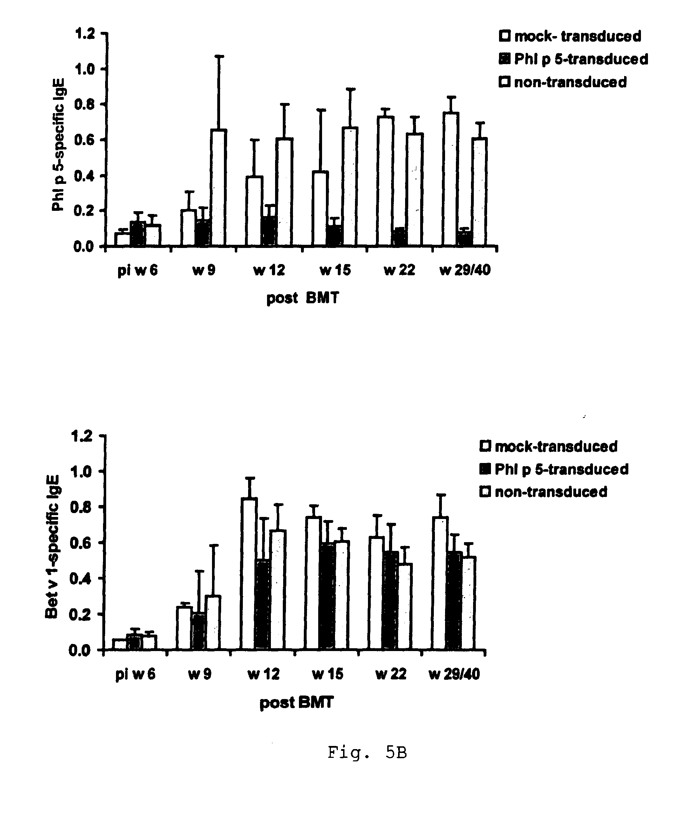

Fig. 5 shows the absence of detectable Phl p 5-specific antibody levels in the serum of Phl p 5-chimeras. Allergen-specific (Phl p 5 and Bet v 1) IgG1 (A) and allergen-specific IgE (B) levels were measured in serum by ELISA at multiple time points post-BMT. Pre-immune level (pi) was determined prior to the first sensitisation. Samples of two independent experiments were pooled. One experiment was terminated at week 40, the second experiment at week 29. Groups of mice transplanted with Phl p 5-transduced (n=10), mock-transduced (n=3) BM and sensitised non-transduced mice (n=10) were analysed. In mice transplanted with Phl p 5-transduced BM no Phl p 5-specific IgG1 and IgE could be detected at any time point throughout follow-up. Antibody development to control allergen Bet v 1 occurred unimpeded, demonstrating the specificity of the induced state of tolerance.

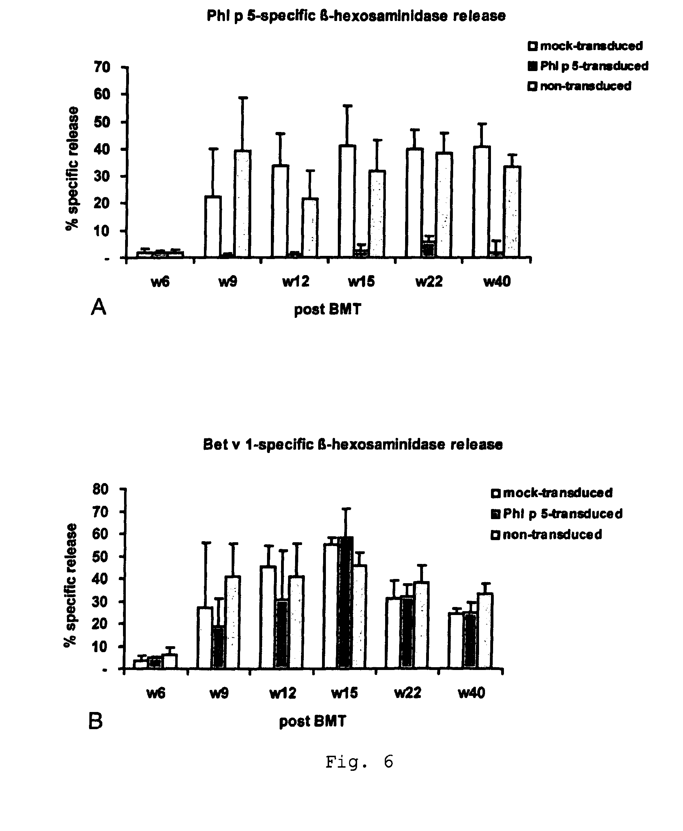

Fig. 6 shows the basophil degranulation assay in Phl p 5, chimeras. A rat basophil leukaemia (RBL) assay measuring allergen-specific β-hexosaminidase release was performed at several weeks post-BMT, in groups described in Figure legend 5. In Phl p 5 chimeras (n=3) no specific basophil degranulation could be detected, whereas Bet v 1-specific β-hexosaminidase release was similar to control groups (mock-transduced group n=3; non-transduced group n=5).

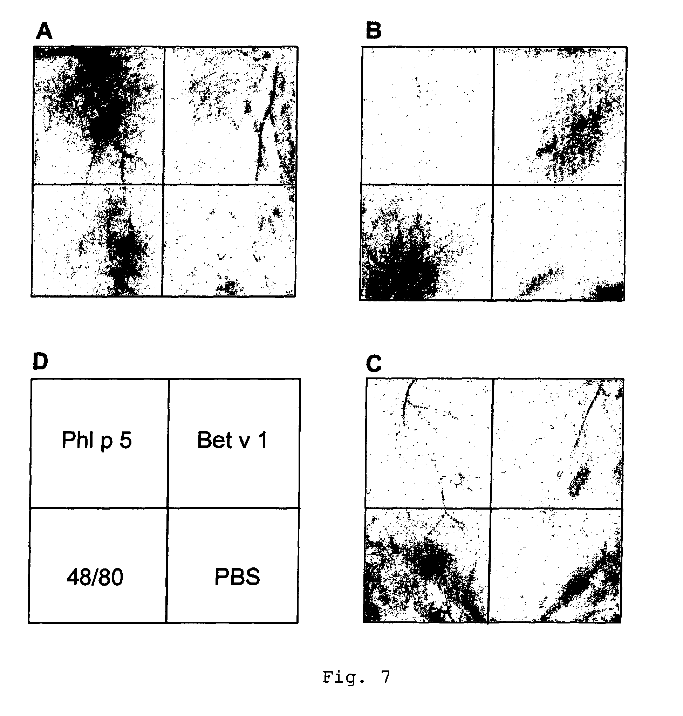

Fig. 7 shows the tolerance towards Phl p 5 in skin prick test in Phl p 5 chimeras. Representative skin sections are shown of non-transduced mice immunised with Phl p 5 and Bet v 1 (A) and Phl p 5 chimeras immunised with Phl p 5 and Bet v 1 (B). As negative control, naive Balb/c mice were injected intradermally with PBS only (C). Skin reactions to rPhl p 5, rBet v 1 and the mast cell-degranulating compound 48/80 and PBS were measured at the inside of the abdominal skin 20 minutes after intradermal injection. (D) shows the intradermal injection scheme.

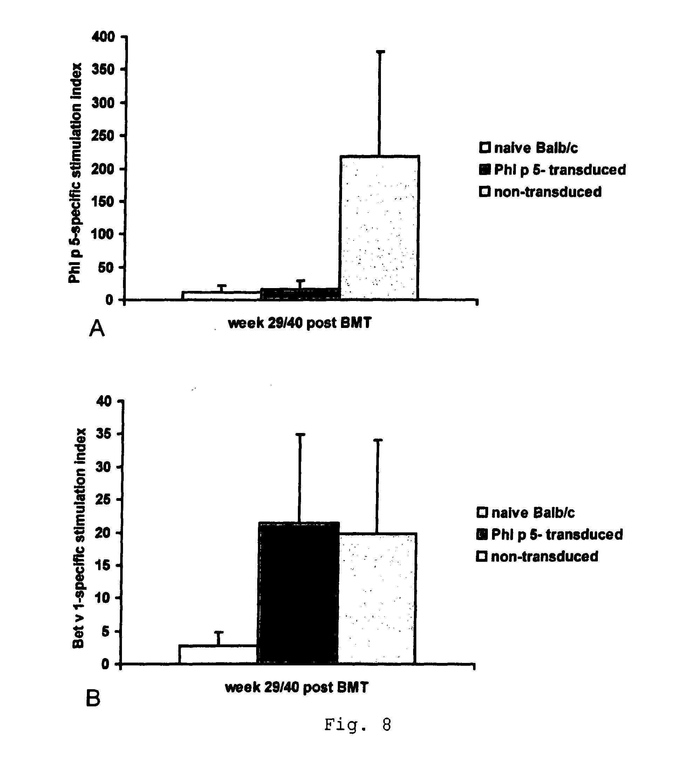

Fig. 8 shows the prevention of Phl p 5-specific T-cell sensitisation in Phl p 5 chimeras. Lymphoproliferation of spleen cells derived from mice of different groups was determined in vitro 29 or 40 weeks post-transplant (shown as SI = stimulation index, in relation to reactivity to medium). (A) Spleen cells were stimulated with rPhl p 5, (B) and rBet v 1, 2µg/ml each. Phl p5-transduced chimeras show a dramatic reduction of proliferation in response to stimulation with Phl p 5, compared to immunised Balb/c mice, whereas both groups show strong proliferation in response to Bet v 1.

### Example 1: Production of recombinant retroviruses integrating fusion genes for membrane-bound or secreted allergen into primary mammalian bone marrow cells

### a)construction of retroviral vectors encoding for recombinant retroviruses carrying membrane-anchored and secreted Phl p 5 and GFP.

To generate retroviral vectors with a secreted Phl p 5 molecule, the original Phl p 5 signal sequence was replaced by the signal sequence (S) of the k light chain of murine immunoglobulin (pDisplay, Invitrogen) and fused to the full length Phl p 5 (Vrtala et al. J Immunol 151(9):4773-4781,1993) and eGFP (vector pEGFP-C1, Clontech), using overlapping PCR technique (Ho et al. Gene 77:51-59, 1989) (Fig. 1A). To generate membrane-anchored Phl p 5, a transmembrane domain (TMD) of human platelet-derived growth factor (pDisplay, Invitrogen) was fused additionally to the signal sequence and Phl p 5 or eGFP (Fig. 1B). Phl p 5 fusion gene restriction sites NcoI at the 5' end and BamHI site at the 3' end and eGFP fusion gene restriction sites NcoI at the 5'end and BglII site at the 3' end were inserted by the following primers (SEQ ID No.'s in parentheses):

| | | | |
|---|---|---|---|
| S-Phl p 5: | S: | SP NcoI forw: | 5′-GGCGC;CATGGAGACAGACACACTCCTG-3′ (1) NcoI |
| | | SP Phl p 5 rev2: | 5'-GTAACCGAGATCGGCGTCACCAGTGGA-3' (2) |
| | Phl p 5: | Phl p 5 SP fw2: | 5'-ACTGGTGACGCCGATCTCGGTTAC-3' (3) |
| | | Phl p 5 * BamHI rev: | 5′-GCCG;GATCCTCAGACTTTGTAGCCACC-3′ (4) BamHI |
| S-Phl p 5-TMD | S: | SP NcoI forw: | 5'-GGCGC;CATGGAGACAGACACACTCCTG-3' (1) NcoI |
| | | SP Phl p 5 rev2: | 5'-GTAACCGAGATCGGCGTCACCAGTGGA-3' (2) |
| | Phl p 5: | Phl p 5 SP fw2: | 5'-ACTGGTGACGCCGATCTCGGTTAC-3' (3) |
| | | Phl p 5 TMD rev.2 | 5'-GCCCACAGCGACTTTGTAGCCACC-3' (5) |
| | TMD: | TMD Phl p 5 forw | 5'-TACAAAGTCGCTGTGGGC-3' (6) |
| | | TMD BamHI rev.: | 5'-GGCG;GATCCTAACGTGGCTTCTTCTG-3'  (7) BamHI |
| S-eGFP: | S: | SP NcoI forw: | 5′-GGCGC;CATGGAGACAGACACACTCCTG-3′  (1) NcoI |
| | eGFP: | SP EGFP rev.: | 5'-CTTGCTCACGTCACCAGT-3' (8) |
| | | EGFP SP fw2: | 5'-ACTGGTGACGTGAGCAAGGGCGAG-3' (9) |
| | | EGFP*BglII rev.: | 5'-GGCA;GATCTCTAACGTGGCTTCTT-3 (10) BglII |
| S-eGFP-TMD | S: | SP NcoI forw: | 5'-GGCGC;CATGGAGACAGACACACTCCTG-3'  (1) NcoI |
| | eGFP: | SP EGFP rev.: | 5'-CTTGCTCACGTCACCAGT-3' (8) |
| | | EGFP SP fw2: | 5'-ACTGGTGACGTGAGCAAGGGCGAG-3' (9) |
| | TMD: | EGFP TMD rev.2: | 5'-GCCCACAGCTCTAGATCCGGTGAA-3' (11) |
| | | TMD EGFP forw.: | 5'-GGATCTAGAGCTGTGGGC-3' (12) |
| | | TMD Bgl II rev.: | 5'-GGCA;GATCTCTAACGTGGCTTCTT-3' (10) BglII |

Fused fragments were cloned into acceptor vector pST-Blue 1 (Novagen). Positive inserts were subcloned into the retroviral vector pMMP (kindly provided by John Iacomini, Boston), resulting in pMMP-Phl p 5, pMMP-eGFP (Fig. 1A), pMMP-Phl p 5-TM and pMMP-eGFP-TM (Fig. 1B). Positive clones were confirmed by double-strand sequencing.

### b) Production of recombinant pseudotyped retroviruses carrying membrane-anchored Phl p 5 and GFP

Production of recombinant retroviruses was performed by transient transfection of 293 T cells (kindly provided by John Iacomini, Boston) with pMMP-Phl p 5 -TM (or pMMP-eGFP-TM), pMD.G and pMLV, using the calcium-phosphate precipitation method (Pear et al., Proc. Natl. Acad. Sci. USA 90:8392-8396, 1993). The MMP retroviral vector is a derivative of MFG (Riviere et al. Proc Natl Acad Sci USA 92 (15): 6733-6737, 1995) and contains the myeloproliferative sarcoma virus (MPSV) long terminal repeat promoter-enhancer elements. These transcriptional elements have been shown to allow expression in hematopoietic cell lineages (Bowtell et al. Mol Biol Med 4(4):229-50.,1987). Vector pMD.G encodes for Vesicular stomatitis virus G (VSV-G) envelope protein to produce VSV-G/retroviral pseudotypes (Ory et al.Proc Natl Acad Sci USA 93(21):11400-11406, 1996). For transfection, 4.5x10⁶ cells were plated in 10cm dishes in Dulbecco minimum essential medium (DMEM) (GIBCO, Invitrogen) containing 10% fetal calf serum (FCS). Four hours before transfection media were exchanged with Isocove's modified DMEM media (GIBCO, Invitrogen) containing 10% FCS and 25mM HEPES (MP Biomedicals, Eschwege, Germany). One plate was transfected with pMMP-Phl p 5-TM (or pMMP-eGFP-TM) (10µg), pMD.G (5µg) and pMLV (7.5µg) respectively. 12 to 16 hours after transfection, media were replaced with DMEM containing 10% FCS and 10mM HEPES. Viral supernatants were collected 72 hours after transfection, filtered and concentrated by ultracentrifugation (Beckman ultracentrifuge). Titers of viral particles were determined by infection of NIH 3T3 cells and analysed by flow cytometry. An Epics XL-MCL flow cytometer (Beckman Coulter, IL Alliance, Austria) was used for acquisition and EXPO32 ADC Software, Applied Cytometry Systems (West Sheffield, UK), was used for analysis of flow cytometric data. To identify surface expression of Phl p 5 a rabbit-polyclonal antiserum against full-length recombinant Phl p 5 (rPhl p 5) was purified with a Protein G column (Pierce) according to the manufacturer's instructions. Purified antibody was biotinylated and titered in Phl p 5-expressing 293T cells.

### Example 2: Induction of allergen-specific tolerance

### a)Experimental design

The aim of the example is to establish a protocol for tolerance induction in type 1 allergy through transplantation of genetically modified syngeneic hematopoietic cells. Donor Balb/c mice were treated with 5-FU at day 7 before BM isolation (Bodine et al.Exp.Hematol 19: 206-212, 1991). BMC (bone marrow cells) were isolated and cultured as described below and treated several times with allergen-integrating virus particles. Recipient Balb/c mice were lethally irradiated (8 Gy) and treated with anti-CD4 and anti-CD8 monoclonal antibodies. Immediately after bone marrow transplantation (BMT), recipients received MR1 (anti-CD40L monoclonal antibody). Mice were injected repeatedly with recombinant Phl p 5 and Bet v 1 post-BMT. Tolerance was determined by a series of in vivo and in vitro assays. To determine the presence of allergen-transduced leukocytes (i.e. molecular chimerism) mice were bled several times and chimerism was determined by flow cytometry (Fig. 4).

Age-matched female Balb/c mice were obtained from Charles River Laboratories (Germany), housed under specific pathogen-free conditions and were used between 8-12 weeks of age for experiments. In the table below the protocol for one typical experiment of Phl p 5-transduced group of Balb/c mice is shown.

| **TBI d-1** | **BMC post cult.** | **Retrovirus** | **rPhl p5 and rBet v1** | **n** |
|---|---|---|---|---|
| Balb/c 8 Gy | 2-4 x10⁶ | VSV-Phl p 5-TM | w 6, 9, 12, 22 | 3 - 7 |

As control, groups with non-transduced Balb/c mice, which were sensitised at weeks 6, 9, 12 and 22 with rPhl p 5 and rBet v 1, as well as groups with either mock-transduced (retroviral production with empty pMMP vector) or VSV-eGFP-TM-transduced BM, were treated like the Phl p 5-transduced mice described in the table above.

### b)Transduction of primary bone marrow cells with VSV-Phl p 5-TM and transplantation in pretreated Balb/c mice

BMC from mice treated 7 days previously with 5-fluorouracil (5-FU; 150mg/kg) were harvested, and isolated BMC were cultured in tissue culture plates coated with Retronectin (Takara Biochemicals, Shiga, Japan) according to the manufacturer's instructions. BMC were cultivated in DMEM (GIBCO, Invitrogen) containing 15% FCS and cytokines to achieve a final concentration of 100ng/ml human interleukin-6 (IL-6; R & D Systems, USA), 100ng/ml recombinant mouse stem cell factor (SCF; Biosource International, USA), 50ng/ml recombinant mouse thrombopoietin (TPO; R & D Systems), and 50ng/ml recombinant mouse Flt-3 ligand (R & D Systems) and 0.1 % Gentamicin (MP Biomedicals, Germany). Transductions were performed at 37°C with 5% CO₂ for 96 hours. BMC were cultured at a density of 4x10⁶ cells/ml and infected with virus particles at a multiplicity of infection (MOI) of 1 to 5. 24 hours later transductions of cells with the same amount of virus particles were repeated. 48 hours later, cells were recovered from plates and centrifuged (Heraeus, 1000 rpm, RT, 5 min) and resuspended in BM media, cytokine-cocktail and infected with virus particles like described before. 24 hours later, cells were recovered from plates, pelleted and resuspended in M199 media (Sigma), 0.02U/ml DNAse (Sigma), 0.08% Gentamicin (MP Biomedicals, Eschwege, Germany) and 10mM HEPES (MP Biomedicals, Germany). Cells were counted and the surface expression of Phl p 5 or GFP was determined by flow cytometry (Fig. 3).

### c)Transplantation of Phl p 5-transduced BM cells in pretreated Balb/c mice

After transduction, 2 to 4x10⁶ BMC were injected i.v. into myeloablated (i.e. lethally irradiated) Balb/c mice. One day before transplantation mice were injected with 0.5 mg anti-CD4 (GK1.5) and anti-CD8 (2.43) antibodies intraperitoneally. Immediately after transplantation, mice were injected with 0.5mg anti-CD40L (MR1) (antibodies were purchased from Bioexpress, West Lebanon, NH, USA).

### Example 3: Stable long-term chimerism of retrovirally-encoded allergen in bone marrow-derived cells in vivo

Recipients of transduced BM were examined for expression of Phl p 5 by flow cytometry. All tested leukocyte lineages are expressing high levels of Phl p 5 for the length of follow-up (>38 weeks). Chimerism reached up to approx. 75%, with typical ranges of 20-40% (Fig. 4). White blood cells were stained with fluorescein isothiocyanate (FITC)-conjugated antibodies against CD4, CD8, B220, Mac-1 (Becton Dickinson, USA) and biotinylated-Phl p 5 developed with phycoerythrin-streptavidin (PEA, Becton Dickinson, USA). Two-colour flow cytometry (excluding dead cells by propidium iodide staining) was used to distinguish Phl p 5 transduced and host cells, and chimerism was calculated as percentage of Phl p 5 positive cells as described by Tomita et al. (Blood 83:939-948, 1994)

### Example 4: Tolerance towards allergens established through molecular chimerism

### a)Phl p 5 molecular chimerism specifically inhibits development of Phl p 5-specific antibodies

Six, 9, 12 and 22 weeks after BMT mice were sensitised with two allergens in order to test specificity, rPhl p 5 and rBet v 1 (5µg /mouse) (Biomay, Austria) were adsorbed to Al(OH)₃ (Alu-Gel-S, Serva, Germany). One day before immunisation mice were bled and serum was obtained. Sera were stored at -20°C until analysis. To analyse if Phl p 5-transduced mice were tolerant toward Phl p 5, antigen-specific IgG1 and IgE serum levels were measured by ELISA (Vrtala et al., J Immunol 160 (12):6137-6144,1998) (Fig. 5A and Fig. 5B). Recombinant Phl p 5 and rBet v 1 (5µg/ml) were coated to 96 well plates (Nunc, Maxisorp, Denmark) and incubated with mouse sera (1:500 for IgG1; 1:20 for IgE ELISA). Bound IgG1 or IgE were detected with rat anti-mouse IgG1 (1:1000) or rat anti-mouse IgE (1:1000) (Pharmingen, San Diego, CA) and with HRP-labelled donkey anti-rat antiserum (1:2000) (Amersham, Buckinghamshire, UK). Colour reactions were measured in ELISA-reader (Wallac, Perkin Elmer, Austria) at 405nm minus 490nm wavelength. Sera of mock-transduced (viral supernatants produced as described before with empty pMMP vector) and untreated sensibilised Balb/c mice showed high Phl p 5-specific IgG1 and IgE levels in contrast to the Phl p 5-transduced mice. All mice transplanted with Phl p 5-transduced BMC demonstrated an absence of detectable levels of Phl p 5-specific IgG1 and IgE, but showed high levels of Bet v 1-specific IgG1 and IgE, indicating that they are specifically tolerant towards Phl p 5. Similar data were obtained in two independent experiments.

### b)Prevention of allergen-specific IgE-dependent basophil degranulation in Phl p 5-tolerant mice

To investigate whether tolerance at the effector level was achieved, a rat basophil leukaemia (RBL) assays were performed. RBL-2H3 cells were plated in 96 well tissue-culture plates (4x10⁴cells/well), incubated for 24h at 37° and 5% CO₂. Cells were incubated with mouse sera (1:50 diluted) of Phl p 5-tolerant mice, mock-transduced mice and non-transduced sensitised Balb/c mice. Preimmune sera and sera after each allergen injection were incubated with RBL cells for 2 hours at 37°C. Supernatants were removed and the cell layer was washed with 2x Tyrode's buffer (137 mM NaCl, 2.7 mM KCl, 0.5mM MgCl₂, 1.8mM CaCl₂, 0.4mM NaH₂PO₄, 5.6mM D-glucose, 12mM NaHCO₃, 10mM HEPES and 0.1% w/v BSA, pH 7.2). Preloaded cells were stimulated with rPhl p 5 of rBet v 1 (0.03µg per well) for 30min at 37°C. For positive control cells were lysed with 1% Triton X-100. The supernatants were analysed for β-hexosaminidase activity by incubation with 80µM 4-methylumbelliferyl-N-acetyl-β-D-glucosamide (Sigma-Aldrich, Austria) in citrate buffer (0.1M, pH4.5) for 1 hour at 37°C. The reaction was stopped by addition of 100µl glycine buffer (0.2M glycine, 0.2M NaCl, pH 10.7) and the fluorescence was measured at λₑₓ: 360/λₑₘ: 465nm using a fluorescence microplate reader (Wallac, Perkin Elmer, Austria). Results are reported as fluorescence units and percentage of total β-hexosaminidase released after lysis of cells with 1% Triton X-100.

As shown in Fig. 6 no basophil degranulation could be observed in the sera of Phl p 5-chimeric mice after stimulation of RBL cells with rPhl p 5. Mock-transduced Balb/c mice showed similar β-hexosaminidase release like non-transduced sensitised Balb/c mice. In contrast, sera of Phl p 5-chimeric mice showed basophil degranulation upon stimulation with Bet v 1 indicating that transplantation of Phl p 5-transduced BMC induced allergen-specific tolerance.

### c) Tolerance towards Phl 5 in skin rick test

To test tolerance in a cutaneous type I hypersensitivity reaction, skin prick tests were performed. Thirty or forty weeks after BMT, mice were injected into the tail vein with 100µl of 0.5% Evans blue (Sigma, USA). Subsequently, 30µl of Phl p 5 and Bet v 1 (0.5µg/ml each, diluted in PBS) were injected intradermally into the shaved abdominal skin. As positive control, the mast cell-degranulating compound 48/80 (20µg/ml, Sigma) was injected intradermally and PBS as negative control. Twenty minutes after injection, mice were sacrificed and the blue colour intensity of the reaction was compared with the individual positive control on the inverted skin. In Fig. 7 the inverted abdominal skin of one representative non-transduced immunised Balb/c mouse like described above (A) shows positive reactions with Bet v 1 as well as Phl p 5. Phl p 5-chimeric mice show a positive reaction with Bet v 1 but not with Phl p 5 (B). Naive Balb/c mice did not show any positive reaction with allergens (C). Figure 7D shows the injection-scheme of recombinant allergens and controls.

### d) Dramatically reduced allergen-specific T-cell roliferation

To test T-cell tolerance additionally to B-cell tolerance, T-cell proliferation assays were performed. At the time mice were killed (29 or 40 weeks after BMT), spleens of age-matched naive Balb/c mice (n=4), Phl p 5-transduced (n=6) and non-transduced (n=7) sensitised mice were isolated. Spleen cells were cultured at a concentration of 2x10⁵ cells/well in 96 roundbottom plates (Nunc, Denmark) in RPMI 1640 medium (Biochrome AG, Germany) at 37°C and 5% CO₂ and stimulated with concanavalin A (Con A; 0.5µg/well, Sigma), rPhl p 5 (2µg/well) and rBet v 1 (2µg/well). On day 5 cultures were pulsed with 0.5µCi/well tritiated thymidin (Amersham) for 16 hours and harvested. The proliferative response was measured by scintillation counting. The ratio of the mean proliferation with antigen (cpm) and with medium control values (cpm) was calculated (stimulation index [SI]). Almost no proliferation could be detected in Phl p 5 chimeras in contrast to non-transduced sensitised Balb/c mice. Bet v 1 stimulated lymphocyte proliferation was similar in Phl p 5 chimeras compared to non-transduced sensitised mice (Fig. 8).

In this model, it could be shown that transplantation of modified syngeneic hematopoietic cells induces molecular chimerism and lasting robust tolerance in type I allergy. This leads to T-cell tolerance shown in lymphocyte proliferation assays, B-cell tolerance (no allergen-specific IgE and IgG1 production in chimeric mice). Importantly, tolerance at the effector cell level was also established, as determined by lack of basophil/mast cell degranulation in vitro and in vivo.

## Claims

1. A method for producing a hematopoietic cell expressing at least one polypeptide derived from at least one allergen by introducing nucleic acids or a DNA molecule encoding said at least one polypeptide.

2. The method according to claim 1, **characterised in that** the hematopoietic cell is selected from the group consisting of monocyte, macrophage, neutrophil, basophil, hemopoietic stem cell, eosinophil, T-cell, B-cell, NK-cell and dendritic cell.

3. The method according to claim 1 or 2, **characterised in that** the DNA molecule encoding said at least one polypeptide is comprised in a DNA vector.

4. The method according to claim 3, **characterised in that** the DNA vector is a viral, preferably retroviral, or a plasmid vector.

5. The method according to claim 3 or 4, **characterised in that** the vector is transiently introduced in the hematopoietic cell.

6. The method according to any one of claims 1 to 5, **characterised in that** the allergen is selected from the group consisting of Phl p 1, Phl p 2, Phl p 5, Phl p 6, Der p 1, Der p 2, Der p 5, Der p 7, Der p 21, Fel d 1, Bet v 1, Ole e 1, Par j 2, Can f 1 and Can f 2.

7. The method according to any one of claims 1 to 6, **characterised in that** the allergen derivative is hypoallergenic.

8. The method according to any one of claims 1 to 7, **characterised in that** the at least one polypeptide is fused to a secretion signal sequence, a membrane anchoring domain or transmembrane domain.

9. The method according to any one of claims 1 to 8, **characterised in that** the DNA molecule is introduced into the hematopoietic cell by chemical methods, preferably by using cationic lipids and cationic polymers, physical methods, preferably particle bombardment, micro-injection or electroporation, viral methods by interaction of the viral envelope with cell surface receptors or abundant phospholipids.

10. A mammalian viral Vector DNA comprising at least one nucleic acid molecule which encodes for a polypeptide derived from an allergen.

11. A vector according to claim 10, **characterised in that** the allergen is selected from the group consisting of Phl p 1, Phl p 2, Phl p 5, Phl p 6, Der p 1, Der p 2, Der p 5, Der p 7, Der p 21, Fel d 1, Bet v 1, Ole e 1, Par j 2, Can f 1 and Can f 2.

12. The vector according to claim 10 or 11, **characterised in that** the allergen derivative is hypoallergenic.

13. The vector according to any one of claims 10 to 12, **characterised in that** the at least one polypeptide is fused to a secretion signal sequence, membrane anchoring domain and/or transmembrane domain.

14. The vector according to any one of claims 10 to 13, **characterised in that** the vector comprises long terminal repeats (LTR's) of preferably moloney murine leukaemia retrovirus or long terminal repeat promoter-enhancer elements of myeloproliferative sarcoma virus (MPSV), a promoter, preferably albumin promoter or cytomegalovirus (CMV) promoter, an origin of replication, preferably of EBV or SV40, or human chromosomal S/MAR.

15. Hematopoietic cell having a cell membrane and expressing at least one polypeptide derived from at least one allergen.

16. Cell according to claim 15, **characterised in that** the at least one polypeptide is fused to a membrane anchoring domain or a transmembrane domain.

17. Cell according to claim 15 or 16, **characterised in that** the at least one polypeptide is bound to the extracellular side of the cell membrane.

18. Cell according to any one of claims 15 to 17 comprising a mammalian viral vector DNA according to any one of claims 10 to 14.

19. Cell according to any one of claims 15 to 18, **characterised in that** the hematopoietic cell is selected from the group consisting of monocyte, macrophage, neutrophil, basophil, hemopoietic stem cell, eosinophil, T-cell, B-cell, NK-cell and dendritic cell.

20. Use of a vector DNA according to any one of claims 10 to 14 or a hematopoietic cell according to any one of claims 15 to 19 for manufacturing a medicament for the treatment or prevention of allergy.

21. A pharmaceutical formulation comprising a vector DNA according to any one of claims 10 to 14 or a hematopoietic cell according to any one of claims 15 to 19.
